(19) Europäisches Patentamt
European Patent Office
Office européen des brevets

(11) Veröffentlichungsnummer : **0 151 744 B1**

(12) # EUROPÄISCHE PATENTSCHRIFT

(45) Veröffentlichungstag der Patentschrift : **26.04.89**

(21) Anmeldenummer : **84115215.0**

(22) Anmeldetag : **12.12.84**

(51) Int. Cl.⁴ : **C 07 D233/70**, C 07 D235/02, C 07 D401/04, C 07 D413/04, C 07 D413/06, A 01 N 43/50

(54) **1-Acylimidazolinone, Verfahren zu ihrer Herstellung und ihre Verwendung in der Landwirtschaft.**

(30) Priorität : **20.12.83 DE 3345901**

(43) Veröffentlichungstag der Anmeldung : **21.08.85 Patentblatt 85/34**

(45) Bekanntmachung des Hinweises auf die Patenterteilung : **26.04.89 Patentblatt 89/17**

(84) Benannte Vertragsstaaten : **AT BE CH DE FR GB IT LI NL SE**

(56) Entgegenhaltungen :
**EP–A– 0 041 623**
**EP–A– 0 095 105**
**DE–A– 2 833 274**
**GB–A– 2 011 876**

(73) Patentinhaber : **HOECHST AKTIENGESELLSCHAFT**
**Postfach 80 03 20**
**D-6230 Frankfurt am Main 80 (DE)**

(72) Erfinder : **Schmierer, Roland, Dr.**
**An der Weinleite 5a**
**D-8901 Todtenweis (DE)**
Erfinder : **Handte, Reinhard, Dr.**
**Theilweg 23**
**D-8901 Gablingen (DE)**
Erfinder : **Mildenberger, Hilmar, Dr.**
**Fasanenstrasse 24**
**D-6233 Kelkheim (Taunus) (DE)**
Erfinder : **Bauer, Klaus, Dr.**
**Kolpingstrasse 7**
**D-6054 Rodgau (DE)**
Erfinder : **Bieringer, Hermann, Dr.**
**Eichenweg 26**
**D-6239 Eppstein/Taunus (DE)**
Erfinder : **Bürstell, Helmut, Dr.**
**Am Hohlacker 65**
**D-6000 Frankfurt am Main 50 (DE)**

**EP 0 151 744 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bereits bekannt geworden, daß Imidazolinone, wie sie beispielsweise in den Deutschen Offenlegungsschriften DE-OS 28 33 274 und DE-OS 31 21 736 beschrieben werden, über herbizide Eigenschaften verfügen. Häufig ist jedoch die Herbizidwirkung der vorgeschlagenen Verbindungen nicht ausreichend, beziehungsweise es treten bei entsprechender Herbizidwirkung Selektivitätsprobleme auf. Das bestehende Bedürfnis nach Problemlösungen bei denen die genannten Nachteile nicht auftreten, wird durch die neuen erfindungsgemäßen Verbindungen der allgemeinen Formel I gelöst.

Gegenstand der vorliegenden Erfindung sind Verbindungen der allgemeinen Formel I,

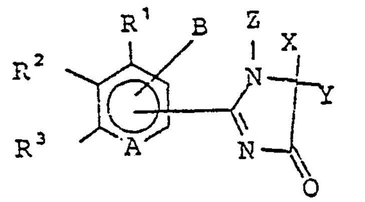

(I)

in welcher

A N oder C—$R^4$;

B 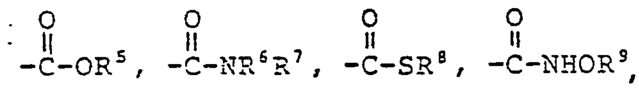

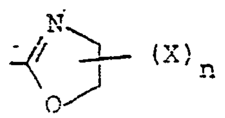 oder 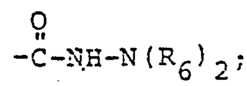;

X $C_1$-bis $C_4$-Alkyl;

Y $C_1$-$C_6$-Alkyl, $C_3$-$C_6$-Cycloalkyl, $C_2$-$C_4$-Alkenyl, $C_2$-$C_4$-Alkinyl, Phenyl oder Benzyl;

X und Y zusammen mit dem Kohlenstoffatom, an dem sie gebunden sind, eine gegebenenfalls durch —$CH_3$ substituierte $C_3$-$C_6$-Spirocycloalkylgruppe;

Z 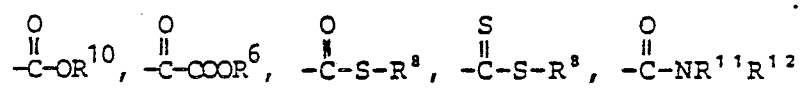 oder

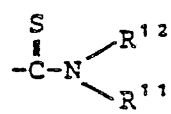;

n 0, 1, 2;

$R^1$, $R^2$, $R^3$ und $R^4$ unabhängig voneinander Wasserstoff, Halogen, $C_1$-$C_4$-Alkyl, $C_1$-$C_6$-Alkoxy, $C_1$-$C_6$-Alkoxy-carbonyl, $C_1$- und $C_2$-Halogenalkyl, Nitro, Cyano, Phenoxy und Phenyl, das gegebenenfalls mit $C_1$-$C_4$-Alkoxy oder Halogen substituiert sein kann, wobei jeweils zwei o-ständige Reste $R^1$, $R^2$, $R^3$ oder $R^4$ auch gemeinsam die Gruppierung —CH=CH—CH=CH— bilden können;

$R^5$ Wasserstoff, $C_1$-$C_{12}$-Alkyl, welches gegebenenfalls bis zu zweimal, vorzugsweise jedoch einfach mit $C_1$-$C_4$-Alkoxy, ($C_1$-$C_4$)-Alkoxy-ethoxy, $C_3$-$C_6$-Cycloalkyl, Benzyloxy, Phenyl, Halogen, Cyano, Hydroxyl, Oxiranyl, Tetrahydrofuryl, ($C_1$-$C_4$)-Alkylamino, ($C_1$-$C_4$)-Dialkylamino, $C_1$-$C_4$-Alkylthio, $C_1$-$C_4$-Alkylsulfinyl, $C_1$-$C_4$-Alkylsulfonyl, Triazolyl oder Imidazolyl substituiert ist; $C_3$-$C_6$-Alkenyl, $C_3$-$C_6$-Halogenalkenyl, $C_3$-$C_6$-Cycloalkyl, $C_5$-$C_6$-Cycloalkenyl, $C_3$-$C_6$-Alkinyl, Benzyl, welches gegebenenfalls im Benzolkern durch Chlor oder Methyl substituiert sein kann; Phenyl, welches gegebenenfalls mit bis zu zwei $C_1$-$C_4$-Alkyl-, Nitro-, Halogen- oder Methoxygruppen substituiert sein kann; die Gruppe

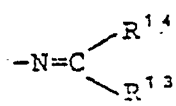

oder ein Kation einer anorganischen oder organischen Base;

$R^6$ Wasserstoff, $C_1$-$C_4$-Alkyl oder Phenyl;

$R^7$ Wasserstoff, $C_1$-$C_6$-Alkyl, welches gegebenenfalls mit einer Hydroxygruppe oder einer $C_1$-$C_3$-

Alkoxygruppe substituiert ist; $C_1$-$C_4$-Alkylsulfonyl, Halogen-$C_1$-$C_4$-alkylsulfonyl; $C_3$-Alkenyl oder $C_3$-Alkinyl;

$R^6$ und $R^7$ gemeinsam mit dem Stickstoffatom einen 5- oder 6gliedrigen Ring, in dem ein Kohlenstoffatom durch Sauerstoff oder Stickstoff ersetzt sein kann und der gegebenenfalls mit bis zu zwei Methylgruppen substituiert ist;

$R^8$ Wasserstoff, $C_1$-$C_6$-Alkyl, Phenyl, Methylphenyl, Halogenphenyl oder ein Kation einer anorganischen oder organischen Base;

$R^9$ Wasserstoff oder $C_1$-$C_6$-Alkyl;

$R^{10}$ einen Rest der für $R^5$ angegebenen Bedeutung, ausgenommen Wasserstoff, Alkylaminoalkyl oder

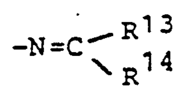

$R^{11}$ Wasserstoff, $C_1$-$C_{12}$-, vorzugsweise $C_1$-$C_{16}$-Alkyl, welches gegebenenfalls mit bis zu drei Halogenen, mit einer $C_1$-$C_4$-Alkoxy-, einer Nitro-, einer Cyano- oder einer Phenylgruppe substituiert sein kann, $C_3$-$C_6$-Cycloalkyl, $C_3$-$C_6$-Alkenyl, $C_3$-$C_6$-Alkinyl, Phenyl, das gegebenenfalls mit bis zu zwei Halogen-, $C_1$-$C_4$-Alkyl-, Trihalogenmethyl-, Nitro-, $C_1$-$C_4$-Alkoxy- oder Cyanogruppen substituiert sein kann, Amino, $C_1$-$C_4$-Alkylamino, Anilino, $C_1$-$C_4$-Dialkylamino, $C_1$-$C_6$-Alkylcarbonyl, Trihalogenacetyl, Benzoyl, Halogenbenzoyl, Methylbenzoyl, Phenylsulfonyl oder Halogenphenylsulfonyl;

$R^{12}$ einen Rest der für $R^6$ angegebenen Bedeutung;

$R^{11}$ und $R^{12}$ gemeinsam mit dem Stickstoffatom einen 5- oder 6gliedrigen Ring, in dem ein Kohlenstoffatom durch Sauerstoff oder Stickstoff ersetzt sein kann und der gegebenenfalls mit bis zu zwei Methylgruppen substituiert ist;

$R^{13}$ und $R^{14}$ unabhängig voneinander Wasserstoff, $C_1$-$C_4$-Alkyl, $C_1$-$C_4$-Alkoxyalkyl, $C_3$-$C_6$-Cycloalkyl oder Phenyl, oder gemeinsam mit dem Kohlenstoffatom, an dem sie gebunden sind, eine $C_5$- oder $C_6$-Cycloalkylgruppe;

bedeuten, sowie deren optische Isomere (falls $X \neq Y$), Säureadditionsverbindungen und N-Oxide (für den Fall, daß A für N steht).

Für Kationen in $R^5$- und/oder $R^8$-Stellung kommen bevorzugt $Na^+$, $K^+$, $1/2\,Mg^{2+}$, $1/2\,Ca^{2+}$, $1/2\,Cu^{2+}$, $1/2\,Fe^{2+}$, $1/2\,Zn^{2+}$, $NH_4^+$ oder organisch substituiertes Ammonium wie $^+NH(C_2H_5)_3$, $^+NH_3CH_2CH_2OH$ oder $^+NH(CH_2CH_2OH)_3$ in Betracht, Säureadditionsverbindungen (bei A = N) werden mit starken Säuren wie HCl oder $H_2SO_4$ gebildet.

Man erhält die erfindungsgemäßen Verbindungen der Formel I, indem man die Verbindungen der Formel II

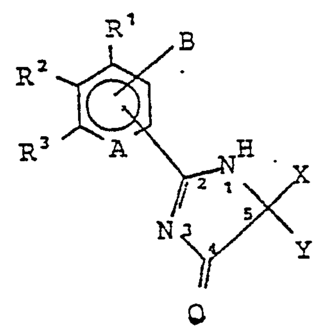

(II)

am Imidazolinring in 1-Stellung acyliert.

Die Verbindungen der Formel II sind an sich bekannt oder lassen sich nach bekannten Verfahren, wie sie in DE-OS 28 33 274 und DE-OS 31 21 736 beschrieben werden, herstellen. Sind weitere acylierbare Gruppierungen im Molekül vorhanden, so können diese zuvor geschützt (z. B. als Ester oder Benzylether) und nach Acylierung mit geeigneten Methoden (z. B. Verseifung, Hydrierung, Umesterung) in die Verbindungen der Formel I umgewandelt werden.

Als Acylierungsmittel geeignet sind zum Beispiel Acylhalogenide in Gegenwart von Basen; Isocyanate oder Isothiocyanate. Auch können aus den Verbindungen II reaktive Zwischenprodukte (wie z. B. Xanthogenate, Chlorcarbonylverbindungen) hergestellt werden, die dann durch z. B. Alkylierung, Alkoholyse, Aminolyse, Hydrazinolyse zu den erfindungsgemäßen Verbindungen nach an sich bekannten Verfahren (Houben-Weyl IX S. 823 ff.) weiter umgesetzt werden können. Die Reaktionstemperaturen für die Acylierungen sind unkritisch. Üblicherweise wird bei Temperaturen von $-10\,°C$ bis $+140\,°C$, vorteilhaft bei $0\,°C$ bis $100\,°C$ in Gegenwart eines inerten Lösemittels gearbeitet. Als Basen für die Umsetzung der Acylhalogenide sind sowohl anorganische, wie Natriumcarbonat, als auch organische, wie z. B. Pyridin oder Triethylamin, geeignet. Bei den Umsetzungen der Isocyanate und Isothiocyanate wirkt sich im allgemeinen der Zusatz von katalytischen Mengen einer tertiären Base vorteilhaft aus.

Die Verbindungen der Formel II sind tautomer, so daß auch die erfindungsgemäßen Verbindungen in einer der beiden Formen Ia/Ib oder als Gemisch von Ia und Ib vorliegen können.

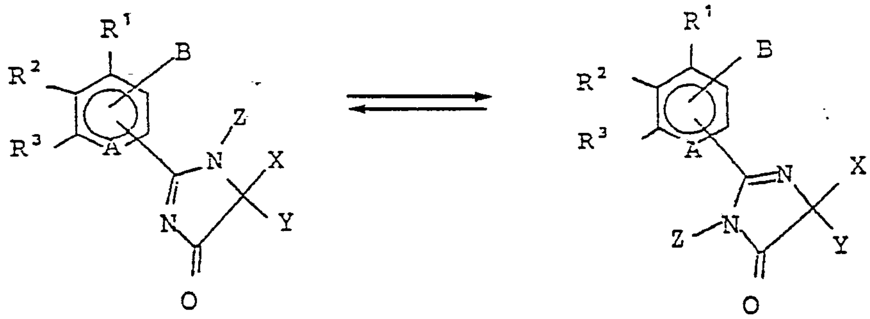

(Ia) (Ib)

Werden für die Synthese der Phenylverbindungen (A = $CR^4$) unsymmetrisch substituierte Phthalsäurederivate eingesetzt, so sind für die Verbindungen der Formel II Isomerengemische möglich (vgl. DE-OS 2 833 274). Die erfindungsgemäßen Verbindungen können dann ebenfalls als Gemisch der beiden Stellungsisomeren vorliegen.

Die vorliegenden erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Wurzelunkräuter werden durch die Wirkstoffe gut erfaßt. Dabei ist es gleichgültig, ob die Substanzen in Vorsaat-Vorauflauf- oder Nachauflaufspritzung ausgebracht werden. Werden die erfindungsgemäßen Verbindungen vor dem Keimen auf die Erdoberfläche appliziert, so wird das Auflaufen der Keimlinge nicht vollständig verhindert. Die Unkräuter wachsen bis zum Keimblattstadium heran, stellen jedoch dann ihr Wachstum ein und sterben schließlich nach drei Wochen vollkommen ab.

Bei einigen Verbindungen tritt bei der Applikation auf die grünen Planzenteile im Nachauflaufverfahren ebenfalls sehr rasch nach der Behandlung ein Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt werden kann. Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja teilweise nur unwesentlich oder gar nicht geschädigt. Einige der erfindungsgemäßen Substanzen besitzen deshalb gegenüber dem Stand der Technik eine wesentlich verbesserte Selektivität bei Kulturpflanzen. Sie eignen sich aus diesen Gründen sehr gut zur Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z. B. durch Auslösen von Desikkation, Abszission und Wuchsstauchung eingesetzt werden. Desweiteren eignen sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösunge, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxyethylierte Fettalkohole, Alkyl- oder Alkylphenylsylfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden : Alkyl-arylsulfonsaure Calciumsalze wie Cadodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen, z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z. B. etwa 10 bis 30 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u. a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,05 und 5 kg/ha.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Nachstehend seien einige Formulierungsbeispiele aufgeführt :

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether, 3 Gewichtsteilen Isotridecanolpolyglykolether (8 AeO) (AeO = Ethylenoxideinheiten) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

Die folgenden Beispiele dienen der weiteren Erläuterung der Erfindung :

## A. Herstellungsbeispiele

### Beispiel 1

2-(2-Ethoxycarbonylphenyl)-5-isopropyl-1-methoxycarbonyl-5-methyl-4-oxo-2-imidazolin

8 g (0,028 mol) 2-(2-Ethoxycarbonylphenyl)-5-isopropyl-5-methyl-4-oxo-2-imidazolin werden in 30 ml abs. Pyridin gelöst und bei Raumtemperatur mit 5,3 g (0,056 mol) Chlorameisensäuremethylester versetzt. Nach 1 h gießt man auf Eis, extrahiert 2mal mit je 100 ml Toluol, wäscht die organische Phase 2mal mit Wasser, trocknet und dampft ein. Man erhält 8,7 g (90 % d. Th.) 2-(2-Ethoxycarbonylphenyl)-5-isopropyl-1-methoxycarbonyl-5-methyl-4-oxo-2-imidazolin, ein farbloses Öl.

$^1$H-NMR (60 MHz, $CDCl_3$) $\delta$ = 0,98, 1,09 (2d, J = 7 Hz, $—CH—(CH_3)_2$, je 3H) ; 1,28 (t, J = 7 Hz, $—OCH_2CH_3$, 3H) ; 1,43 (s, $—CH_3$, 3H) ; 2,17 (h, J = 7 Hz, $CH—(CH_3)_2$, 1H) ; 3,63 (s, $OCH_3$, 3H) ; 4,25 (q, J = 7 Hz, $O—CH_2—$, 2H) ; 7,3-8,0 ppm (m, 4H, Phenyl).

### Beispiel 2

2-(2-Ethoxycarbonylphenyl)-1-N-ethylcarbamoyl-5-isopropyl-5-methyl-4-oxo-2-imidazolin

9 g (0,031 mol) 2-(2-Ethoxycarbonylphenyl)-5-isopropyl-5-methyl-4-oxo-2-imidazolin werden in 30 ml abs. Methylenchlorid gelöst und bei Raumtemperatur mit 2,7 g (0,038 mol) Ethylisocyanat und 0,5 ml DBU

(DBU = 1.8-Diaza-Bicyclo-[5.4.0]-undec-7-en) versetzt. Nach 1 h Reaktionszeit wird auf Eis gegeben, die organische Phase 2mal mit Wasser gewaschen, getrocknet und eingedampft. Man erhält 11,0 g (98 % d. Th.) 2-(2-Ethoxycarbonylphenyl)-1-N-methylcarbamoyl-5-isopropyl-5-methyl-4-oxo-2-imidazolin, farblose Tafeln vom Fp 85-87 °C.

Beispiel 3

2-(2-Butoxycarbonylphenyl)-1-N',N'-dimethylcarbamoyl-5-isopropyl-5-methyl-4-oxo-2-imidazolin

Zu einer Lösung von 3,85 g (0,039 mol) Phosgen in 25 ml Chlorbenzol tropft man bei Raumtemperatur ein Gemisch aus 10 g (0,032 mol) 2-(2-Butoxycarbonylphenyl)-5-isopropyl-5-methyl-4-oxo-2-imidazolin, 3 g (0,038 mol) Pyridin und 30 ml Methylchlorid zu. Man läßt 1 h bei Raumtemperatur rühren, kühlt auf 5 °C ab und tropft bei dieser Temperatur 4,4 g (0,073 mol) N,N-Dimethylhydrazin zu. Man läßt auf Ramtemperatur erwärmen, gießt auf Wasser, wäscht die organische Phase noch 2mal mit je 100 ml Wasser, trocknet und dampft ein. Nach chromatografischer Reinigung (Kieselgel, Laufmittel Petrolether-Essigester 7 : 3) erhält man 7,8 g (61 % d. Th.) 2-(2-Butoxycarbonylphenyl)-1-N',N'-dimethylcarbamoyl-5-isopropyl-5-methyl-4-oxo-2-imidazolin, ein hellgelbes Öl.

$^1$H-NMR (60 MHz, $CDCl_3$) δ = 0,97, 1,10 (2d, J = 7 Hz, je 3H, $—CH(CH_3)_2$) ; 1,43 (s, 3H, $CH_3$) ; 0,85-2,3 (sh, 8H, $CH_2—CH_2—CH_3$, $—CH(CH_3)_2$) ; 2,53 (s, 6H, $N(CH_3)_2$) ; 4,17 (t, J = 7 Hz, 2H, $—OCH_2$) ; 7,3-8,2 ppm (m, 4H, Phenyl) ; 8,90 ppm (s, 1H, NH).

In analoger Weise werden die Beispiele der Tabelle 1 hergestellt.

(Siehe Tabelle 1 Seite 7 ff.)

Tabelle 1

Verbindungen der allgemeinen Formel I

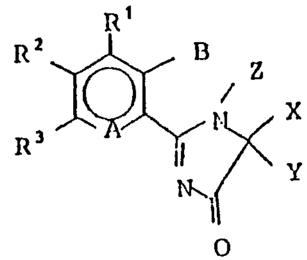

| Beispiel Nr. | $R^1$ | $R^2$ | $R^3$ | A | B | X | Y | Z | Fp[°C] |
|---|---|---|---|---|---|---|---|---|---|
| 4 | H | H | H | CH | $CONH_2$ | $CH_3$ | $CH_3$ | $-C(=S)-SCH_3$ | Oel |
| 5 | " | " | " | " | COOH | " | " | 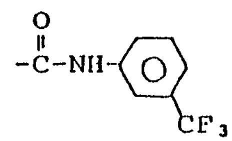 | " |
| 6 | " | " | " | N | $COOCH_3$ | " | $C_2H_5$ | $-C(=O)-NH-n-C_8H_{17}$ | " |
| 7 | " | " | " | " | " | " | $CH(CH_3)_2$ | $-C(=O)-NH-NHCH_3$ | " |
| 8 | " | " | " | " | " | " | " | $-C(=O)-NH-CH_3$ | 91-3 |

Tabelle 1 (Fortsetzung)

| Beispiel Nr. | $R^1$ | $R^2$ | $R^3$ | A | B | X | Y | Z | Fp [°C] |
|---|---|---|---|---|---|---|---|---|---|
| 9 | H | H | H | CH | $COOCH_3$ | $CH_3$ | $CH(CH_3)_2$ | $-\overset{O}{\overset{\|}{C}}-NH-CH_3$ | Harz |
| 10 | " | " | " | " | " | " | " | $-\overset{O}{\overset{\|}{C}}-NH-C_2H_5$ | Oel |
| 11 | " | " | " | " | " | " | " | $-\overset{S}{\overset{\|}{C}}-NH-C_2H_5$ | |
| 12 | " | " | " | " | " | " | " | $-\overset{O}{\overset{\|}{C}}-NH-CH_2-CH=CH_2$ | |
| 13 | " | " | " | " | " | " | " | $-\overset{O}{\overset{\|}{C}}-NH-C_4H_9$ | Oel |
| 14 | " | " | " | " | " | " | " | $-\overset{O}{\overset{\|}{C}}-N(CH_3)_2$ | Oel |
| 15 | " | " | " | " | " | " | " | 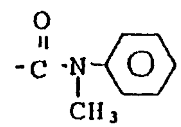 | Oel |
| 16* | F | " | " | " | " | " | " | 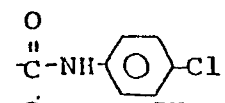 | Oel |
| 17* | " | " | Cl | " | " | " | " | 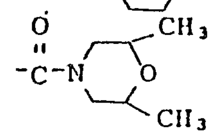 | " |

Tabelle 1 (Fortsetzung)

| Beispiel Nr. | $R^1$ | $R^2$ | $R^3$ | A | B | X | Y | Z | Fp [°C] |
|---|---|---|---|---|---|---|---|---|---|
| 18 * | F | H | $CH_3$ | CH | $COOCH_3$ | $CH_3$ | $CH(CH_3)_2$ | $-\overset{O}{\overset{\|}{C}}-NH_2$ | Harz |
| 19 * | " | " | $COOCH_3$ | " | " | " | " | " | Oel |
| 20 | Cl | Cl | Cl | CCl | " | " | " | $-\overset{O}{\overset{\|}{C}}-NH-CH_3$ | " |
| 21 | H | H | H | CH | " | " | " | $-\overset{O}{\overset{\|}{C}}-SNa$ | Harz |
| 22 | " | " | " | " | " | " | " | $-\overset{O}{\overset{\|}{C}}-S-C_2H_5$ | Oel |
| 23 | " | " | " | " | " | " | " | $-\overset{O}{\overset{\|}{C}}-OCH_3$ | " |
| 24 | " | " | " | " | " | " | " | $-\overset{O}{\overset{\|}{C}}-O-nC_4H_9$ | " |
| 25 | " | " | " | " | " | " | " | $-\overset{O}{\overset{\|}{C}}-NH-NH_2$ | |
| 26 | " | " | " | " | " | " | " | 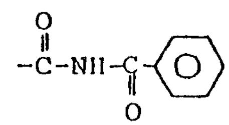 | |

Tabelle 1 (Fortsetzung)

| Beispiel Nr. | $R^1$ | $R^2$ | $R^3$ | A | B | X | Y | Z | Fp[°C] |
|---|---|---|---|---|---|---|---|---|---|
| 27 | H | H | H | CH | $COOC_2H_5$ | $CH_3$ | $-CH(CH_3)_2$ | $-\overset{O}{\overset{\|}{C}}-NH-CH_3$ | 90-2 |
| 28 | " | " | " | " | " | " | " | $-\overset{O}{\overset{\|}{C}}-OC_2H_5$ | Oel |
| 29 | " | " | " | " | " | " | " | 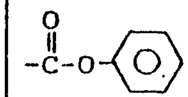 | " |
| 30 | " | " | " | " | $COOnC_4H_9$ | " | " | $-\overset{O}{\overset{\|}{C}}-NH-CH_3$ | 73-5 |
| 31 | " | " | " | " | " | " | " | $-\overset{O}{\overset{\|}{C}}-NHC_2H_5$ | Oel |
| 32 | " | " | " | " | " | " | " | 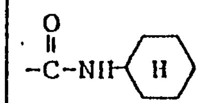 | " |
| 33 | " | " | " | " | " | " | " | 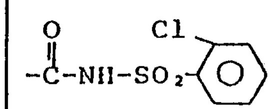 | Harz |
| 34 | " | " | " | " | 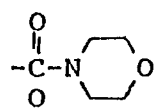 | " | 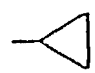 | $-\overset{S}{\overset{\|}{C}}-NH_2$ | Oel |
| 35 | " | " | " | " | $-\overset{O}{\overset{\|}{C}}-S-nC_4H_9$ | " | $-CH_2CH(CH_3)_2$ | 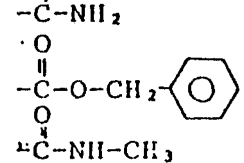 | " |
| 36*) | $NO_2$ | " | " | " | " | " | " | $-\overset{O}{\overset{\|}{C}}-NH-CH_3$ | " |

Tabelle 1 (Fortsetzung)

| Beispiel Nr. | $R^1$ | $R^2$ | $R^3$ | A | B | X | Y | Z | Fp [°C] |
|---|---|---|---|---|---|---|---|---|---|
| 37 | H | $CH_3$ | H | CH | $-COO(CH_2)_2-OCH_3$ | $C_2H_5$ | $C_2H_5$ | $-\overset{O}{\overset{\|}{C}}-N(C_2H_5)_2$ | |
| 38 | " | " | " | " | $-COO(CH_2)_2-Cl$ | " | " | $-\overset{O}{\overset{\|}{C}}-NH-\underset{O}{\underset{\|}{C}}-CCl_3$ | |
| 39 | " | $-CH=CH-CH=CH-$ | | " | $-COONa$ | $CH_3$ | $-CH(CH_3)_2$ | $-\overset{O}{\overset{\|}{C}}-NHCH_3$ | |
| 40 | " | " | | " | $-COO(CH_2)_2-N(CH_3)_2$ | " | " | $-\overset{O}{\overset{\|}{C}}\cdot SCH_3$ | |
| 41 | " | " | | N | $-COOH$ | " | $C_2H_5$ | $-\overset{O}{\overset{\|}{C}}-N(CH_3)_2$ | Hydrochlorid |
| 42 | " | " | | " | $-COOCH_2CH=CH_2$ | $C_2H_5$ | " | 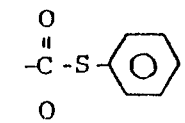 | |
| 43 | " | H | CN | CH | $-COO$ 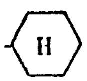 | " | $CH(CH_3)_2$ | $-\overset{O}{\overset{\|}{C}}-NHCH_2CH_2OCH_3$ | |
| 44 | " | " | H | " | $-CONHOCH_3$ | $-\underset{CH_3}{\underset{\|}{CH}}CH_2CH_2CH_2CH_2-$ | | 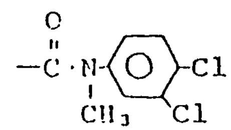 | |

Tabelle 1 (Fortsetzung)

| Beispiel Nr. | $R^1$ | $R^2$ | $R^3$ | A | B | X | Y | Z | Fp [°C] |
|---|---|---|---|---|---|---|---|---|---|
| 45 | H | H | H | CH | 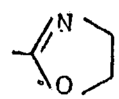 | $-CHCH_2CH_2CH_2CH_2-$<br>$CH_3$ | | O<br>‖<br>$-C-S-C_3H_7$ | |
| 46 | " | " | " | " | 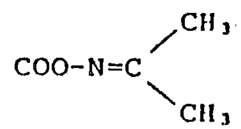 | " | | O<br>‖<br>$-C-O-CH_2-CH_2-Cl$ | |
| 47 | " | " | " | " | $COOCH_3$ | $CH_3$ | $C_2H_5$ | $-CONHCH_3$ | 97-101 |
| 48 | " | " | " | " | $COOC_2H_5$ | " | " | " | 81 |
| 49 | " | " | " | " | " | " | " | $-CONHC_2H_5$ | " |
| 50 | " | " | " | N | $COOCH_3$ | " | $CH(CH_3)_2$ | " | " |
| 51 | " | " | " | " | " | " | " | $-COOCH_3$ | " |
| 52 | " | $-CH=CH-CH=CH-$ | | CH | " | " | " | $-CONHCH_3$ | 136-41 |

Tabelle 1 (Fortsetzung)

| Beispiel Nr. | $R^1$ | $R^2$ | $R^3$ | A | B | X | Y | Z | Fp [°C] |
|---|---|---|---|---|---|---|---|---|---|
| 53 | H | H | H | CH | $COOCH_3$ | $CH_3$ | $CH(CH_3)_2$ | $-CONH-CH(CH_3)_2$ | Öl |
| 55 | " | " | " | " | " | " | " | 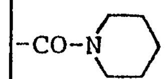 | Öl |
| 56 | " | " | " | " | " | " | " | 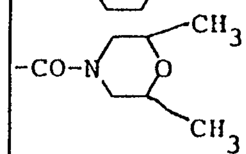 | Öl |
| 57 | " | " | " | " | " | " | " | 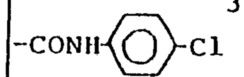 | Öl |
| 58 | " | " | " | " | " | " | " | $-CONHC(CH_3)(CN)CH(CH_3)_2$ | 68-74 |
| 59 | " | " | " | " | " | " | " | $-COCOOCH_3$ | 92-3 |
| 60 | " | " | " | " | $COOC_2H_5$ | " | " | $-CONH-n-C_4H_9$ | Öl |
| 61 | " | " | " | " | $COO-n-C_4H_9$ | " | " | $-CONH_2$ | Öl |
| 62 * | " | $CH_3$ | " | " | $COOCH_3$ | " | " | $-CONHCH_3$ | Öl |

Tabelle 1 (Fortsetzung)

| Beispiel Nr. | $R^1$ | $R^2$ | $R^3$ | A | B | X | Y | Z | Fp [°C] |
|---|---|---|---|---|---|---|---|---|---|
| 63 * | H | $CH_3$ | H | CH | $COOCH_3$ | $CH_3$ | $CH(CH_3)_2$ | $-CONHC_2H_5$ | Öl |
| 64 * | " | " | " | " | " | " | " | $-COCOOCH_3$ | " |
| 65 * | " | " | " | " | " | " | " | $-COOCH_3$ | " |
| 66 * | " | " | " | " | " | " | " | -COO Phenyl | " |
| 67 * | " | " | " | " | " | " | " | $-COS-n-C_6H_{13}$ | " |
| 68 * | " | " | " | " | " | " | " | $-CONH-N(CH_3)_2$ | " |
| 69 * | " | " | " | " | " | " | " | $-CONH-CH(CH_3)_2$ | " |
| 70 * | " | " | " | " | " | " | " | -CONH 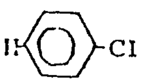 -Cl | " |
| 71 | " | " | " | " | " | " | $CH(CH_3)C_2H_5$ | $CONHCH_3$ | " |
| 72 | " | Cl | Cl | " | " | " | $CH(CH_3)_2$ | " | " |

Tabelle 1 (Fortsetzung)

| Beispiel Nr. | $R^1$ | $R^2$ | $R^3$ | A | B | X | Y | Z | Fp [°C] |
|---|---|---|---|---|---|---|---|---|---|
| 73 * | H | H | H | $CCH_3$ | $COOCH_3$ | $CH_3$ | $CH(CH_3)_2$ | $CONHCH_3$ | Öl |
| 74 | " | " | " | N | $COOCH_2-S-CH_3$ | " | " | " | " |
| 75 | " | $-CH=CH-CH=CH-$ | | " | $COOCH_3$ | " | " | " | " |
| 76 | " | " | | " | " | " | " | $COOCH_3$ | " |
| 77 | " | " | | " | COOH | " | " | $CONHCH_3$ | " |
| 78 | " | H | H | " | $COO^- H_3N^+CH(CH_3)_2$ | " | " | " | " |

* im Gemisch mit der stellungsisomeren Verbindung.

B. Biologische Beispiele

Prüfung auf herbizide Wirkung

Die Schädigung der Unkrautpflanzen bzw. die Kulturpflanzenverträglichkeit wurde in einem Schlüssel von 0-5 bonitiert.
Dabei bedeutet

0 = ohne Wirkung (Schaden)
1 = 0- 20 % Wirkung
2 = 20- 40 % Wirkung
3 = 40- 60 % Wirkung
4 = 60- 80 % Wirkung
5 = 80-100 % Wirkung

1. Wirkung gegen Unkräuter

Samen bzw. Rhizomstücke mono- und dikotyler Unkräuter wurden in Lehmerde in Plastiktöpfen (∅ 9 cm) ausgelegt und mit Erde abgedeckt. Die als benetzbare Pulver bzw. als Emulsionskonzentrate formulierten erfindungsgemäßen Verbindungen wurden in Form wäßriger Suspensionen bzw. Emulsionen auf die Erdoberfläche appliziert. Die Wasseraufwandmenge pro Topf entsprach dabei umgerechnet 600 l/ha. Nach der Behandlung wurden die Versuchstöpfe im Gewächshaus aufgestellt und die Versuchspflanzen unter guten Wachstumsbedingungen (Temperatur : 23 ± 1 °C ; rel. Luftfeuchte 60-80 %) kultiviert. Nach ca. 3 Wochen wurde die Pflanzenschädigung visuell bonitiert. Als Vergleich dienten dabei unbehandelte Kontrollen.

Die erfindungsgemäßen Verbindungen wiesen eine zum Teil ausgezeichnete herbizide Wirksamkeit gegen wirtschaftlich bedeutende mono- und dikotyle Schadpflanzen auf.

Tabelle 2

Wirksamkeit im Vorauflauf gegen

| Beisp. Nr. | ALM | POA | AMR | SIA |
|---|---|---|---|---|
| 1 | 5/5 | 5/3 | 5/5/5 | 5/5 |
| 2 | 5/5 | 5 | 5/5/5 | 5/4 |
| 3 | 5/5 | 5 | 5/5/5 | 5/4 |
| 8 | 5/5/5 | 5/5/5 | 5/5/5 | 5/5/5 |
| 9 | 5/5/4 | 5/5 | 5/5/4 | 5/5/4 |
| 10 | 5/5 | 5/5 | 5/5/5 | 5/5 |
| 15 | 5 | 4 | 5 | 5 |
| 22 | 5/5 | 3/3 | 5/5 | 5/5 |
| 23 | 5/5 | 5/5 | 5/5/5 | 5/5 |
| 24 | 5/5/4 | 5/5 | 5/5/4 | 5/5 |
| 27 | 5/5 | 5 | 5/5/5 | 5/5 |
| 28 | 5/5 | 5 | 5/5/5 | 5/5 |
| 29 | 5/5 | 4/3 | 5/5/5 | 5/4 |
| 30 | 5 | 5 | 5/5 | 5/5 |
| 31 | 5/4 | 5 | 5/5 | 5/4 |
| 32 | 4 | | 5 | 4 |
| 33 | 5 | 4 | 5/5 | 5/5 |
| 47 | 5/5 | 5/5 | 5/4 | 5/4 |
| 48 | 4 | 4 | 5/5 | 5/5 |
| 49 | 4/4 | 4 | 5/5 | 5/5 |

Tabelle 2 (Fortsetzung)

| Beisp. Nr. | ALM | POA | AMR | SIA |
|---|---|---|---|---|
| 50 | 5/5/5 | 5/5/5 | 5/5/5 | 5/5/5 |
| 51 | 5/5/5 | 5/5/5 | 5/5/5 | 5/5/5 |
| 52 | 5 | | 4/4 | 5/4 |
| 53 | 5/5 | 5/5 | 5/5/4 | 5/5/4 |
| 55 | 5 | 4 | 5 | 5 |
| 56 | 4 | 4 | 5/4 | 4 |
| 57 | 5/5 | 5/5 | 5/5/5 | 5/5/4 |
| 58 | 5/5 | 5/5/4 | 5/5 | 5/5 |
| 59 | 5/5 | 5/5 | 5/5 | 5/5 |
| 60 | 5/4 | 4 | 5/5/5 | 5/4 |
| 61 | 5/5 | 5/5 | 5/5/4 | 5/5 |
| 62 | 5/5/5 | 5/4 | 5/4 | 5/5/4 |
| 63 | 5/5/5 | 5 | 5/4 | 5/5 |
| 64 | 5/5/5 | 5/4 | 5/4 | 5/5/4 |
| 65 | 5/5/5 | 5/4 | 5/4 | 5/5 |
| 66 | 5/5/5 | 5/4 | 5 | 5/5 |
| | **LOM** | **ECG** | **STM** | **SIA** |
| 68 | 4 | 4 | 5 | 5 |
| 69 | 4 | | 4 | 5 |
| 70 | 5 | 4 | 5 | 5 |

In ähnlicher Weise werden verschiedene Unkräuter in Töpfen im Gewächshaus bis zum 3-6-Blattstadium herangezogen und dann im Nachauflaufverfahren mit den erfindungsgemäßen Verbindungen (formuliert als Spritzpulver) behandelt. 4 Wochen später wurden die Versuchspflanzen im Vergleich zu unbehandelten Kontrollpflanzen visuell bonitiert, indem die Schädigung geschätzt wurde.

Die erfindungsgemäßen Verbindungen erwiesen sich auch in diesem Versuch als gut wirksam :

Tabelle 3

Wirksamkeit im Nachauflauf gegen

| Beisp. Nr. | ALM | POA | AMR | SIA |
|---|---|---|---|---|
| 1 | 5 | | | 5/4 |
| 3 | | | 5 | 4 |
| 8 | 5/5 | 5/5/4 | 5/4 | 5/5 |
| 9 | 4 | 4 | 5 | 4 |
| 10 | | | | 5/4 |
| 22 | | | | 5 |
| 23 | | | | 5/4 |
| 24 | | | | 4 |
| 27 | 4 | | | 4 |
| 28 | | | 5 | |

Tabelle 3 (Fortsetzung)

| Beisp. Nr. | ALM | POA | AMR | SIA |
|---|---|---|---|---|
| 29 | 4 | | | 4 |
| 47 | | 3 | 5 | 4 |
| 50 | 5/5 | 5/5/4 | 5/5 | 5/5/4 |
| 51 | 5/5 | 5/5/5 | 5/5/4 | 5/5/5 |
| 57 | | | 5 | 5 |
| 58 | 4 | | 5/4 | 5/5 |
| 59 | 5 | | | 5/5/4 |
| 62 | 5/4 | | | 5/4/4 |
| 63 | 5 | | | 5/5 |
| 64 | 5/4 | | | 5/5/4 |
| 65 | 5/4 | | 4 | 5/5/4 |
| 66 | 4/4 | 3 | 3 | 5/4 |
| 68 | 5/4 | | 4 | 5/5/4 |
| 69 | 5/4 | | 4 | 5/5/4 |
| 70 | 5/4 | | 4 | 5/5/4 |

Legende :

Testpflanzen : ALM = Alopecurus myosuroides
POA = Poa annua
AMR = Amaranthus retroflexus
SIA = Sinapis alba

LOM = Lolium multiflorum
ECG = Echinochloa crus-galli
STM = Stellaria media

1 Zahl = Dosierung 2,4 kg/ha — z. B. 5
2 Zahlen = Dosierungen 2,4-0,6 kg/ha — z. B. 5/4
3 Zahlen = Dosierungen 2,4-0,6-0,15 kg/ha — z. B. 5/4/3

**Patentansprüche** (für die Vertragsstaaten : BE, CH, DE, FR, GB, IT, LI, NL, SE)

1. Verbindungen der Formel I

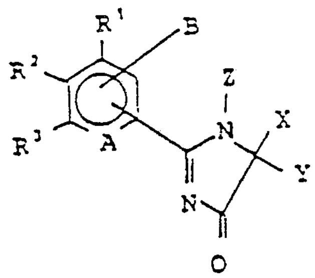

(I)

in welcher

A N oder C-$R^4$ ;

B $-\overset{O}{\overset{\|}{C}}-OR^5$, $-\overset{O}{\overset{\|}{C}}-NR^6R^7$, $-\overset{O}{\overset{\|}{C}}-SR^8$, $-\overset{O}{\overset{\|}{C}}-NHOR^9$,

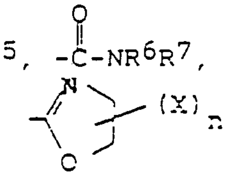 oder $-\overset{O}{\overset{\|}{C}}-NH-N(R_6)_2$ ;

X $C_1$-bis $C_4$-Alkyl ;

Y $C_1$-$C_6$-Alkyl, $C_3$-$C_6$-Cycloalkyl, $C_2$-$C_4$-Alkenyl, $C_2$-$C_4$-Alkinyl, Phenyl oder Benzyl ;

X und Y zusammen mit dem Kohlenstoffatom, an dem sie gebunden sind, eine gegebenenfalls durch —$CH_3$ substituierte $C_3$-$C_6$-Spirocycloalkylgruppe ;

Z 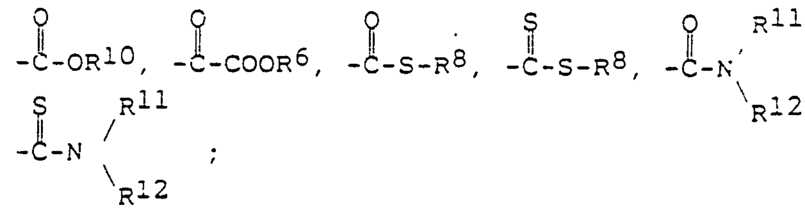 oder ;

n 0, 1, 2

$R^1$, $R^2$, $R^3$ und $R^4$ unabhängig voneinander Wasserstoff, Halogen, $C_1$-$C_4$-Alkyl, $C_1$-$C_6$-Alkoxy, $C_1$-$C_6$-Alkoxycarbonyl, $C_1$- und $C_2$-Halogenalkyl, Nitro, Cyano, Phenoxy und Phenyl, das gegebenenfalls mit $C_1$-$C_4$-Alkoxy oder Halogen substituiert sein kann, wobei jeweils zwei o-ständige Reste $R^1$, $R^2$, $R^3$ oder $R^4$ auch gemeinsam die Gruppierung —CH=CH—CH=CH— bilden können ;

$R^5$ Wasserstoff, $C_1$-$C_{12}$-Alkyl, welches gegebenenfalls bis zu zweimal mit $C_1$-$C_4$-Alkoxy, ($C_1$-$C_4$)-Alkoxyethoxy, $C_3$-$C_6$-Cycloalkyl, Benzyloxy, Phenyl, Halogen, Cyano, Hydroxyl, Oxiranyl, Tetrahydrofuryl, ($C_1$-$C_4$)-Alkylamino, ($C_1$-$C_4$)-Dialkylamino, $C_1$-$C_4$-Alkylthio, $C_1$-$C_4$-Alkylsulfinyl, $C_1$-$C_4$-Alkylsulfonyl, Triazolyl oder Imidazolyl substituiert ist ; $C_3$-$C_6$-Alkenyl, $C_3$-$C_6$-Halogenalkenyl, $C_3$-$C_6$-Cycloalkyl, $C_5$-$C_6$-Cycloalkenyl, $C_3$-$C_6$-Alkinyl, Benzyl, welches gegebenenfalls im Benzolkern durch Chlor oder Methyl substituiert sein kann ; Phenyl ; welches gegebenenfalls mit bis zu zwei $C_1$-$C_4$-Alkyl-, Nitro-, Halogen- oder Methoxygruppen substituiert sein kann ; die Gruppe

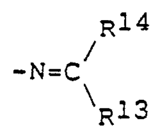

oder ein Kation einer anorganischen oder organischen Base ;

$R^6$ Wasserstoff, $C_1$-$C_4$-Alkyl oder Phenyl ;

$R^7$ Wasserstoff, $C_1$-$C_6$-Alkyl, welches gegebenenfalls mit einer Hydroxygruppe oder einer $C_1$-$C_3$-Alkoxygruppe substituiert ist ; $C_1$-$C_4$-Alkylsulfonyl, Halogen-$C_1$-$C_4$-alkylsulfonyl ; $C_3$-Alkenyl oder $C_3$-Alkinyl ;

$R^6$ und $R^7$ gemeinsam mit dem Stickstoffatom einen 5- oder 6gliedrigen Ring, in dem ein Kohlenstoffatom durch Sauerstoff oder Stickstoff ersetzt sein kann und der gegebenenfalls mit bis zu zwei Methylgruppen substituiert ist ;

$R^8$ Wasserstoff, $C_1$-$C_6$-Alkyl, Phenyl, Methylphenyl, Halogenphenyl oder ein Kation einer anorganischen oder organischen Base ;

$R^9$ Wasserstoff oder $C_1$-$C_6$-Alkyl ;

$R^{10}$ einen rest der für $R^5$ angegebenen Bedeutung, ausgenommen Wasserstoff, Alkylaminoalkyl oder

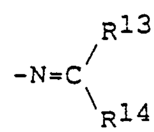

$R^{11}$ Wasserstoff, $C_1$-$C_{12}$-, vorzugsweise $C_1$-$C_6$-Alkyl, welches gegebenenfalls mit bis zu drei Halogenen, mit einer $C_1$-$C_4$-Alkoxy-, einer Nitro-, einer Cyano- oder einer Phenylgruppe substituiert sein kann, $C_3$-$C_6$-Cycloalkyl, $C_3$-$C_6$-Alkenyl, $C_3$-$C_6$-Alkinyl, Phenyl, das gegebenenfalls mit bis zu zwei Halogen-, $C_1$-$C_4$-Alkyl, Trihalogenmethyl-, Nitro-, $C_1$-$C_4$-Alkoxy- oder Cyanogruppen substituiert sein kann, Amino, $C_1$-$C_4$-Alkylamino, Anilino, $C_1$-$C_4$-Dialkylamino, $C_1$-$C_6$-Alkylcarbonyl, Trihalogenacetyl, Benzoyl, Halogenbenzoyl, Methylbenzoyl, Phenylsulfonyl oder Halogenphenylsulfonyl ;

$R^{12}$ einen Rest der für $R^6$ angegebenen Bedeutung ;

$R^{11}$ und $R^{12}$ gemeinsam mit dem Stickstoffatom einen 5- oder 6gliedrigen Ring, in dem ein Kohlenstoffatom durch Sauerstoff oder Stickstoff ersetzt sein kann und der gegebenenfalls mit bis zu zwei Methylgruppen substituiert ist ;

$R^{13}$ und $R^{14}$ unabhängig voneinander Wasserstoff, $C_1$-$C_4$-Alkyl, $C_1$-$C_4$-Alkoxyalkyl, $C_3$-$C_6$-Cycloalkyl oder Phenyl, oder gemeinsam mit dem Kohlenstoffatom, an dem sie gebunden sind, eine $C_5$- oder $C_6$-Cycloalkylgruppe bedeuten ;

sowie deren optische Isomere (falls X ≠ Y), Säureadditionsverbindungen und N-Oxide (für den Fall, daß A für N steht).

2. Verfahren zur Herstellung von Verbindungen der Formel I gemäß Anspruch 1, dadurch gekennzeichnet, daß man Verbindungen der Formel II

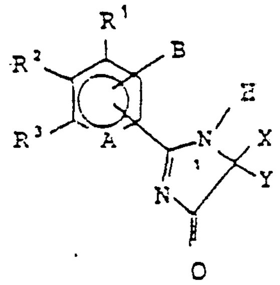

(II)

in 1-Stellung des Imidazolinon-Ringes unter Einführung des Restes Z acyliert.

3. Die Verwendung der Verbindungen der Formel I gemäß Anspruch 1 als Biozide, insbesondere Herbizide und Wachstumsregulatoren.

4. Herbizide und wachstumsregulierende Mittel, dadurch gekennzeichnet, daß sie eine Verbindung der allgemeinen Formel I gemäß Anspruch 1 und 2 enthalten.

5. Verfahren zur Bekämpfung von unerwünschten Pflanzen, dadurch gekennzeichnet, daß man auf die zu behandelnden Flächen eine wirksame Menge einer Verbindung der Formel I gemäß Anspruch 1 aufbringt.

6. Verbindung der Formel

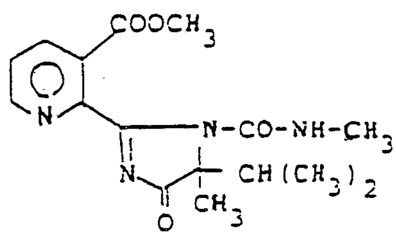

7. Verbindung der Formel

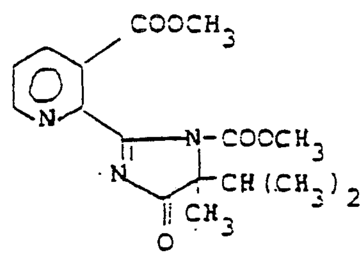

8. Verbindung der Formel

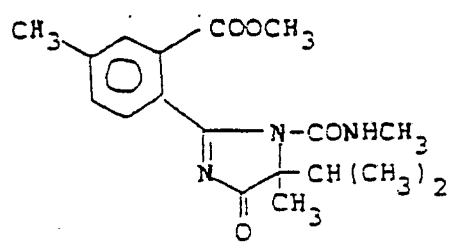

9. Verbindung der Formel

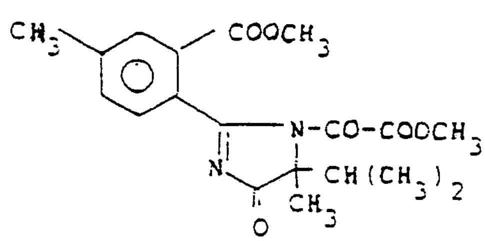

10. Verbindung der Formel

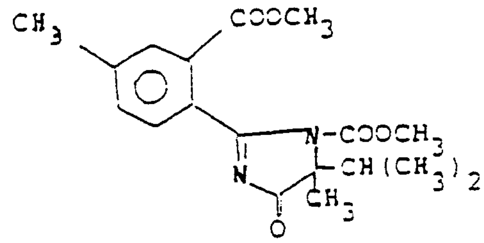

11. Verbindung der Formel

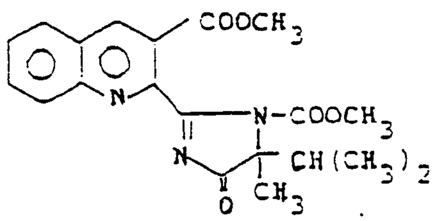

**Patentansprüche** (für den Vertragsstaat AT)

1. Herbizide und wachstumsregulierende Mittel, gekennzeichnet durch einen Gehalt an Verbindungen der Formel I

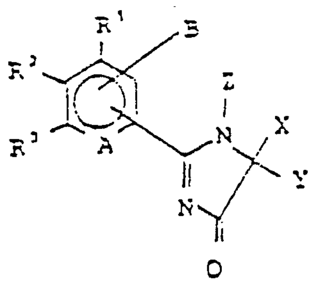

(I)

in welcher

A N oder C-$R^4$ ;

B 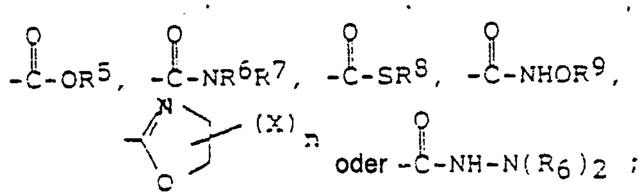

oder ;

X $C_1$- bis $C_4$-Alkyl ;

Y $C_1$-$C_6$-Alkyl, $C_3$-$C_6$-Cycloalkyl, $C_2$-$C_4$-Alkenyl, $C_2$-$C_4$-Alkinyl, Phenyl oder Benzyl ;

X und Y zusammen mit dem Kohlenstoffatom, an dem sie gebunden sind, eine gegebenenfalls durch —$CH_3$ substituierte $C_3$-$C_6$-Spirocycloalkylgruppe ;

Z 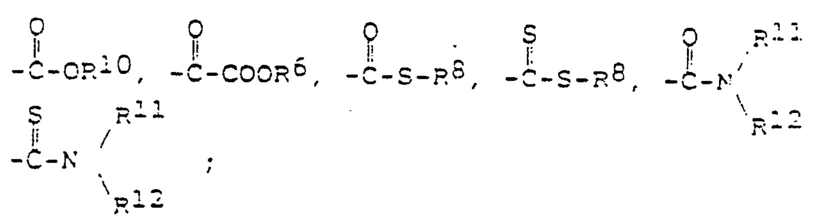

oder ;

n 0, 1, 2

$R^1$, $R^2$, $R^3$ und $R^4$ unabhängig voneinander Wasserstoff, Halogen, $C_1$-$C_4$-Alkyl, $C_1$-$C_6$-Alkoxy, $C_1$-$C_6$-Alkoxycarbonyl, $C_1$- und $C_2$-Halogenalkyl, Nitro, Cyano, Phenoxy und Phenyl, das gegebenenfalls mit $C_1$-$C_4$-Alkoxy oder Halogen substituiert sein kann, wobei jeweils zwei o-ständige Reste $R^1$, $R^2$, $R^3$ oder $R^4$ auch gemeinsam die Gruppierung —CH=CH—CH=CH— bilden können ;

$R^5$ Wasserstoff, $C_1$-$C_{12}$-Alkyl, welches gegebenenfalls bis zu zweimal mit $C_1$-$C_4$-Alkoxy, ($C_1$-$C_4$)-Alkoxyethoxy, $C_3$-$C_6$-Cycloalkyl, Benzyloxy, Phenyl, Halogen, Cyano, Hydroxyl, Oxiranyl, Tetrahydrofuryl, ($C_1$-$C_4$)-Alkylamino, ($C_1$-$C_4$)-Dialkylamino, $C_1$-$C_4$-Alkylthio, $C_1$-$C_4$-Alkylsulfinyl, $C_1$-$C_4$-Alkylsulfonyl, Triazolyl oder Imidazolyl substituiert ist ; $C_3$-$C_6$-Alkenyl, $C_3$-$C_6$-Halogenalkenyl, $C_3$-$C_6$-Cycloalkyl, $C_5$- + $C_6$-Cycloalkenyl, $C_3$-$C_6$-Alkinyl, Benzyl, welches gegebenenfalls im Benzolkern durch Chlor oder Methyl substituiert sein kann, Phenyl, welches gegebenenfalls mit bis zu zwei $C_1$-$C_4$-Alkyl-, Nitro-, Halogen- oder Methoxygruppen substituiert sein kann, die Gruppe

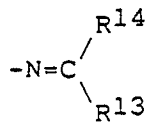

oder ein Kation einer anorganischen oder organischen Base

$R^6$ Wasserstoff, $C_1$-$C_4$-Alkyl oder Phenyl ;

$R^7$ Wasserstoff, $C_1$-$C_6$-Alkyl, welches gegebenenfalls mit einer Hydroxygruppe oder einer $C_1$-$C_3$-Alkoxygruppe substituiert ist ; $C_1$-$C_4$-Alkylsulfonyl, Halogen-$C_1$-$C_4$-alkylsulfonyl, $C_3$-Alkenyl oder $C_3$-Alkinyl ;

$R^6$ und $R^7$ gemeinsam mit dem Stickstoffatom einen 5- oder 6gliedrigen Ring, in dem ein Kohlenstoffatom durch Sauerstoff oder Stickstoff ersetzt sein kann und der gegebenenfalls mit bis zu zwei Methylgruppen substituiert ist ;

$R^8$ Wasserstoff, $C_1$-$C_6$-Alkyl, Phenyl, Methylphenyl, Halogenphenyl oder ein Kation einer anorganischen oder organischen Base ;

$R^9$ Wasserstoff oder $C_1$-$C_6$-Alkyl ;

$R^{10}$ einen Rest der für $R^5$ angegebenen Bedeutung, ausgenommen Wasserstoff, Alkylaminoalkyl oder

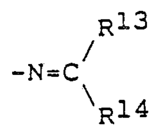

$R^{11}$ Wasserstoff, $C_1$-$C_{12}$-, vorzugsweise $C_1$-$C_6$-Alkyl, welches gegebenenfalls mit bis zu drei Halogenen, mit einer $C_1$-$C_4$-Alkoxy, einer Nitro-, einer Cyano- oder einer Phenylgruppe substituiert sein kann, $C_3$-$C_6$-Cycloalkyl, $C_3$-$C_6$-Alkenyl, $C_3$-$C_6$-Alkinyl, Phenyl, das gegebenenfalls mit bis zu zwei Halogen-, $C_1$-$C_4$-Alkyl, Trihalogenmethyl-, Nitro-, $C_1$-$C_4$-Alkoxy- oder Cyanogruppen substituiert sein kann, Amino, $C_1$-$C_4$-Alkylamino, Anilino, $C_1$-$C_4$-Dialkylamino, $C_1$-$C_6$-Alkylcarbonyl, Trihalogenacetyl, Benzoyl, Halogenbenzoyl, Methylbenzoyl, Phenylsulfonyl oder Halogenphenylsulfonyl ;

$R^{12}$ einen Rest der für $R^6$ angegebenen Bedeutung ;

$R^{11}$ und $R^{12}$ gemeinsam mit dem Stickstoffatom einen 5- oder 6gliedrigen Ring, in dem ein Kohlenstoffatom durch Sauerstoff oder Stickstoff ersetzt sein kann und der gegebenenfalls mit bis zu zwei Methylgruppen substituiert ist ;

$R^{13}$ und $R^{14}$ unabhängig voneinander Wasserstoff, $C_1$-$C_4$-Alkyl, $C_1$-$C_4$-Alkoxyalkyl, $C_3$-$C_6$-Cycloalkyl oder Phenyl, oder gemeinsam mit dem Kohlenstoffatom, an dem sie gebunden sind, eine $C_5$- oder $C_6$-Cycloalkylgruppe bedeuten ;

sowie von deren optischen Isomeren (falls X ≠ Y), Säure-Additionsverbindungen und N-Oxiden (für den Fall, daß A für N steht).

2. Verfahren zur Herstellung von Verbindungen der Formel I gemäß Anspruch 1, dadurch gekennzeichnet, daß man Verbindungen der Formel II

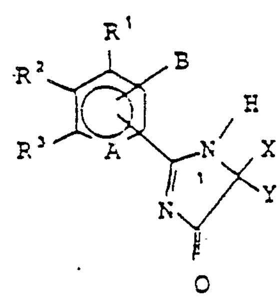

(II)

in 1-Stellung des Imidazolinon-Ringes unter Einführung des Restes Z acyliert.

3. Die Verwendung der Verbindungen der Formel I gemäß Anspruch 1 als Herbizide und Wachstumsregulatoren.

4. Verfahren zur Bekämpfung von unerwünschten Pflanzen, dadurch gekennzeichnet, daß man auf die zu behandelnden Flächen eine wirksame Menge einer Verbindung der Formel I gemäß Anspruch 1 aufbringt.

**Claims** (for the Contracting States : BE, CH, DE, FR, GB, IT, LI, NL, SE)

1. A compound of the formula I

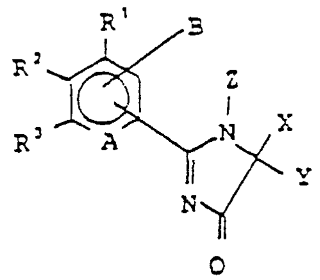
(I)

in which

A denotes N or C—$R^4$ ;

B denotes 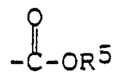, 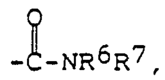, 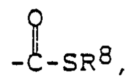, 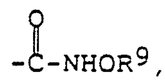, 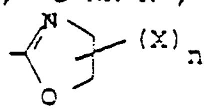 or 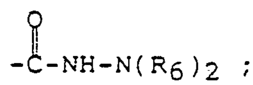 ;

X denotes $C_1$ to $C_4$-alkyl ; and

Y denotes $C_1$-$C_6$-alkyl, $C_3$-$C_6$-cycloalkyl, $C_2$-$C_4$-alkenyl, $C_2$-$C_4$-alkinyl, phenyl or benzyl ; or

X and Y, together with the carbon atom to which they are bonded, denote a $C_3$-$C_6$-spirocycloalkyl group which is optionally substituted by —$CH_3$ ;

Z denotes 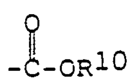, 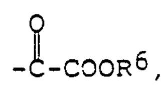, 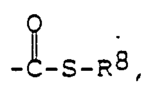, 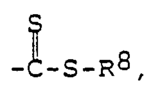, 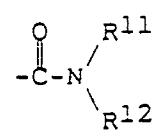 or 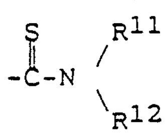 ;

n denotes 0, 1 or 2 ;

$R^1$, $R^2$, $R^3$ and $R^4$ independently of one another denote hydrogen, halogen, $C_1$-$C_4$-alkyl, $C_1$-$C_6$-alkoxy, $C_1$-$C_6$-alkoxycarbonyl, $C_1$- or $C_2$-halogenoalkyl, nitro, cyano, phenoxy or phenyl, which can optionally be substituted by $C_1$-$C_4$-alkoxy or halogen, it being possible for in each case two radicals $R^1$, $R^2$, $R^3$ or $R^4$ in the o-position relative to one another also together to form the grouping —CH=CH—CH=CH— ;

$R^5$ denotes hydrogen, $C_1$-$C_{12}$-alkyl, which is optionally substituted up to twice by $C_1$-$C_4$-alkoxy, ($C_1$-$C_4$)-alkoxyethoxy, $C_3$-$C_6$-cycloalkyl, benzyloxy, phenyl, halogen, cyano, hydroxyl, oxiranyl, tetrahydrofuryl, ($C_1$-$C_4$)-alkylamino, ($C_1$-$C_4$)-dialkylamino, $C_1$-$C_4$-alkylthio, $C_1$-$C_4$-alkylsulfinyl, $C_1$-$C_4$-alkylsulfonyl, triazolyl or imidazolyl ; $C_3$-$C_6$-alkenyl, $C_3$-$C_6$-halogenoalkenyl, $C_3$-$C_6$-cycloalkyl, $C_5$-$C_6$-cycloalkenyl, or $C_3$-$C_6$-alkinyl ; benzyl, which can optionally be substituted in the benzene nucleus by chlorine or methyl ; phenyl which can optionally be substituted by up to two $C_1$-$C_4$-alkyl, nitro, halogen or methoxy groups ; the group

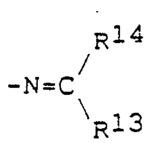

or a cation of an inorganic or organic base ;

$R^6$ denotes hydrogen, $C_1$-$C_4$-alkyl or phenyl ; and

$R^7$ denotes hydrogen, $C_1$-$C_6$-alkyl, which is optionally substituted by a hydroxyl group or a $C_1$-$C_3$-alkoxy group ; $C_1$-$C_4$-alkylsulfonyl or halogeno-$C_1$-$C_4$-alkylsulfonyl ; or $C_3$-alkenyl or $C_3$-alkinyl ; or

$R^6$ and $R^7$, together with the nitrogen atom, denote a 5-membered or 6-membered ring in which one carbon atom can be replaced by oxygen or nitrogen and which is optionally substituted by up to two methyl groups ;

$R^8$ denotes hydrogen, $C_1$-$C_6$-alkyl, phenyl, methylphenyl, halogenophenyl or a cation of an inorganic or organic base ;

$R^9$ denotes hydrogen or $C_1$-$C_6$-alkyl ;

$R^{10}$ denotes a radical having the meaning given for $R^5$, excluding hydrogen, alkylaminoalkyl or

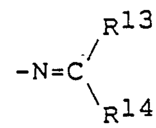

$R^{11}$ denotes hydrogen, $C_1$-$C_{12}$-alkyl, preferably $C_1$-$C_6$-alkyl, which can optionally be substituted by up to three halogen atoms or by a $C_1$-$C_4$-alkoxy, nitro, cyano or phenyl group, $C_3$-$C_6$-cycloalkyl, $C_3$-$C_6$-alkenyl, $C_3$-$C_6$-alkinyl, phenyl, which can optionally be substituted by up to two halogen, $C_1$-$C_4$-alkyl, trihalogenomethyl, nitro, $C_1$-$C_4$-alkoxy or cyano groups, amino, $C_1$-$C_4$-alkylamino, anilino, $C_1$-$C_4$-dialkylamino, $C_1$-$C_6$-alkylcarbonyl, trihalogenoacetyl, benzoyl, halogenobenzoyl, methylbenzoyl, phenylsulfonyl or halogenophenylsulfonyl ; and

$R^{12}$ denotes a radical having the meaning given for $R^6$ ; or

$R^{11}$ and $R^{12}$, together with the nitrogen atom, denote a 5-membered or 6-membered ring, in which one carbon atom can be replaced by oxygen or nitrogen and which is optionally substituted by up to two methyl groups ; and

$R^{13}$ and $R^{14}$ independently of one another denote hydrogen, $C_1$-$C_4$-alkyl, $C_1$-$C_4$-alkoxyalkyl, $C_3$-$C_6$-cycloalkyl or phenyl, or together with the carbon atom to which they are bonded denote a $C_5$- or $C_6$-cycloalkyl group ; and their optical isomers (if $X \neq Y$), acid addition compounds and N-oxides (where A represents N).

2. A process for the preparation of a compound of the formula I as claimed in claim 1, which comprises acylating a compound of the formula II

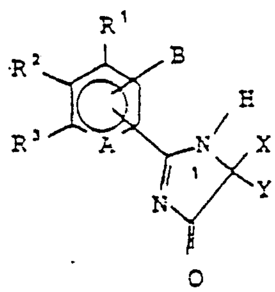

(II)

in the 1-position of the imidazolinone ring, the radical Z being introduced.

3. The use of a compound of the formula I as claimed in claim 1 as a biocide, in particular as a herbicide and growth regulator.

4. A herbicidal and growth-regulating agent, which contains a compound of the formula I as claimed in claims 1 and 2.

5. A method of combating undesirable plants, which comprises applying an effective amount of a compound of the formula I as claimed in claim 1 to the areas to be treated.

6. The compound of the formula

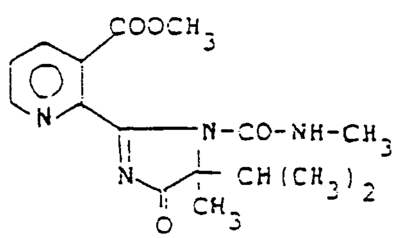

7. The compound of the formula

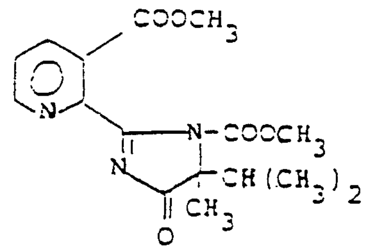

8. The compound of the formula

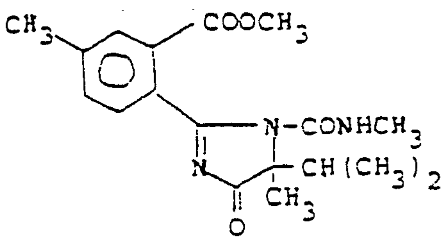

9. The compound of the formula

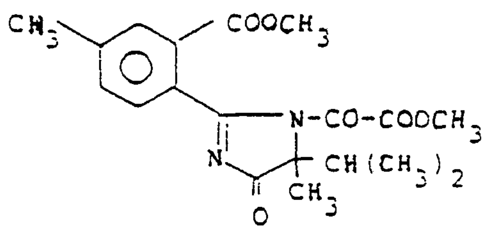

10. The compound of the formula

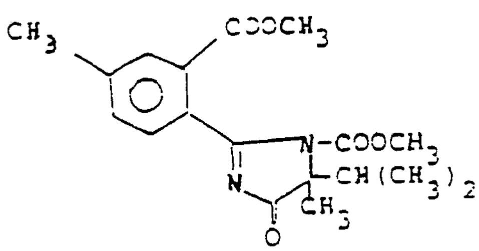

11. The compound of the formula

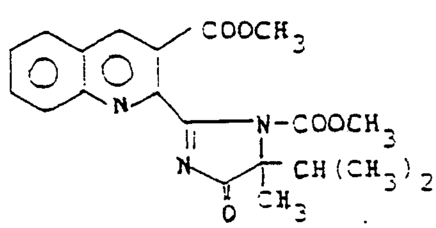

**Claims** (for the Contracting State AT)

1. A herbicide and growth-regulating agent which contains compound of the formula I

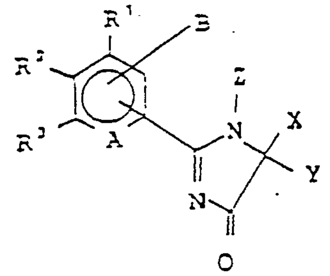

(I)

in which

A denotes N or C—$R^4$;

B denotes 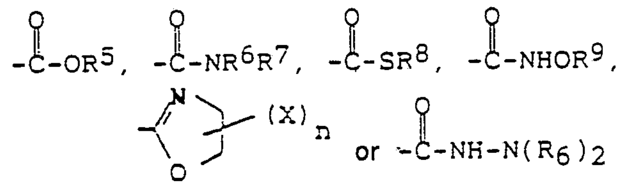 or ;

X denotes $C_1$ to $C_4$-alkyl; and

Y denotes $C_1$-$C_6$-alkyl, $C_3$-$C_6$-cycloalkyl, $C_2$-$C_4$-alkenyl, $C_2$-$C_4$-alkinyl, phenyl or benzyl; or

X and Y, together with the carbon atom to which they are bonded, denote a $C_3$-$C_6$-spirocycloalkyl group which is optionally substituted by —$CH_3$;

Z denotes 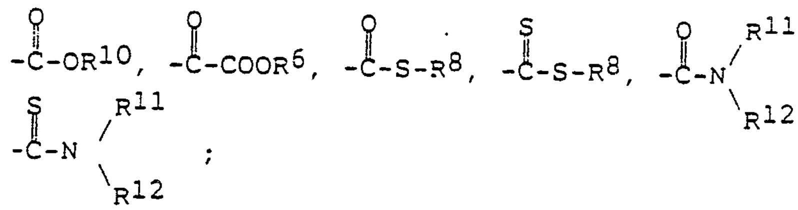 or ;

n denotes 0, 1 or 2;

$R^1$, $R^2$, $R^3$ and $R^4$ independently of one another denote hydrogen, halogen, $C_1$-$C_4$-alkyl, $C_1$-$C_6$-alkoxy, $C_1$-$C_6$-alkoxycarbonyl, $C_1$- or $C_2$-halogenoalkyl, nitro, cyano, phenoxy or phenyl, which can optionally be substituted by $C_1$-$C_4$-alkoxy or halogen, it being possible for in each case two radicals $R^1$, $R^2$, $R^3$ or $R^4$ in the o-position relative to one another also together to form the grouping —CH=CH—CH=CH—;

$R^5$ denotes hydrogen, $C_1$-$C_{12}$-alkyl, which is optionally substituted up to twice by $C_1$-$C_4$-alkoxy, ($C_1$-$C_4$)-alkoxyethoxy, $C_3$-$C_6$-cycloalkyl, benzyloxy, phenyl, halogen, cyano, hydroxyl, oxiranyl, tetrahydrofuryl, ($C_1$-$C_4$)-alkylamino, ($C_1$-$C_4$)-dialkylamino, $C_1$-$C_4$-alkylthio, $C_1$-$C_4$-alkylsulfinyl, $C_1$-$C_4$-alkylsulfonyl, triazolyl or imidazolyl; $C_3$-$C_6$-alkenyl, $C_3$-$C_6$-halogenoalkenyl, $C_3$-$C_6$-cycloalkyl, $C_5$- + $C_6$-cycloalkenyl (sic), or $C_3$-$C_6$-alkinyl; benzyl, which can optionally be substituted in the benzene nucleus by chlorine or methyl; phenyl which can optionally be substituted by up to two $C_1$-$C_4$-alkyl, nitro, halogen or methoxy groups; the group

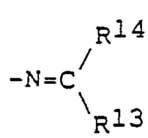

or a cation of an inorganic or organic base;

$R^6$ denotes hydrogen, $C_1$-$C_4$-alkyl or phenyl; and

$R^7$ denotes hydrogen, $C_1$-$C_6$-alkyl, which is optionally substituted by a hydroxyl group or a $C_1$-$C_3$-alkoxy group; $C_1$-$C_4$-alkylsulfonyl or halogeno-$C_1$-$C_4$-alkylsulfonyl; or $C_3$-alkenyl or $C_3$-alkinyl; or

$R^6$ and $R^7$, together with the nitrogen atom, denote a 5-membered or 6-membered ring in which one carbon atom can be replaced by oxygen or nitrogen and which is optionally substituted by up to two methyl groups;

$R^8$ denotes hydrogen, $C_1$-$C_6$-alkyl, phenyl, methylphenyl, halogenophenyl or a cation of an inorganic or organic base;

$R^9$ denotes hydrogen or $C_1$-$C_6$-alkyl;

$R^{10}$ denotes a radical having the meaning given for $R^5$, excluding hydrogen, alkylaminoalkyl or

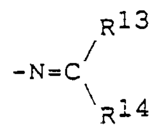

$R^{11}$ denotes hydrogen, $C_1$-$C_{12}$-alkyl, preferably $C_1$-$C_6$-alkyl, which can optionally be substituted by up to three halogen atoms or by a $C_1$-$C_4$-alkoxy, nitro, cyano or phenyl group, $C_3$-$C_6$-cycloalkyl, $C_3$-$C_6$-alkenyl, $C_3$-$C_6$-alkinyl, phenyl, which can optionally be substituted by up to two halogen, $C_1$-$C_4$-alkyl, trihalogenomethyl, nitro, $C_1$-$C_4$-alkoxy or cyano groups, amino, $C_1$-$C_4$-alkylamino, anilino, $C_1$-$C_4$-dialkylamino, $C_1$-$C_6$-alkylcarbonyl, trihalogenoacetyl, benzoyl, halogenobenzoyl, methylbenzoyl, phenylsulfonyl or halogenophenylsulfonyl ; and

$R^{12}$ denotes a radical having the meaning given for $R^6$ ; or

$R^{11}$ and $R^{12}$, together with the nitrogen atom, denote a 5-membered or 6-membered ring, in which one carbon atom can be replaced by oxygen or nitrogen and which is optionally substituted by up to two methyl groups ; and

$R^{13}$ and $R^{14}$ independently of one another denote hydrogen, $C_1$-$C_4$-alkyl, $C_1$-$C_4$-alkoxyalkyl, $C_3$-$C_6$-cycloalkyl or phenyl, or together with the carbon atom to which they are bonded denote a $C_5$- or $C_6$-cycloalkyl group ; and their optical isomers (if $X \neq Y$), acid addition compounds and N-oxides (where A represents N).

2. A process for the preparation of a compound of the formula I as claimed in claim 1, which comprises acylating a compound of the formula II

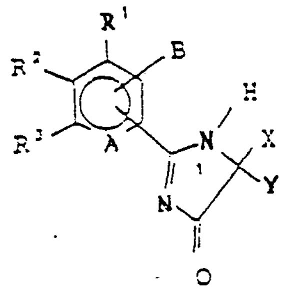

(II)

in the 1-position of the imidazolinone ring, the radical Z being introduced.

3. The use of a compound of the formula I as claimed in claim 1 as a herbicide and growth regulator.

4. A method of combating undesirable plants, which comprises applying an effective amount of a compound of the formula I as claimed in claim I to the areas to be treated.

**Revendications** (pour les Etats contractants : BE, CH, DE, FR, GB, IT, LI, NL, SE)

1. Composés de formule générale I

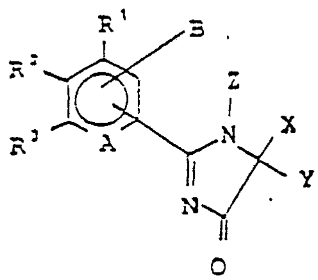

dans laquelle

A est N ou C—$R^4$ ;

B est 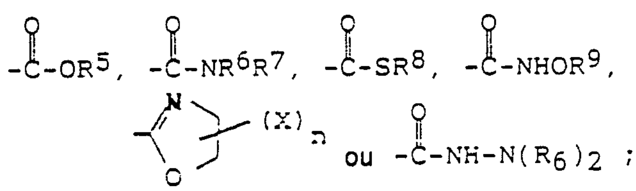

X est un radical alkyle en $C_1$-$C_4$ ;

Y est un radical alkyle en $C_1$-$C_6$, cycloalkyle en $C_3$-$C_6$, alcényle en $C_2$-$C_4$, alcinyle en $C_2$-$C_4$, phényle ou benzyle ;

X et Y forment, avec l'atome de carbone auquel ils sont liés, un groupe spirocycloalkyle en $C_3$-$C_6$ éventuellement substitué par un radical —$CH_3$ ;

Z est 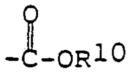, 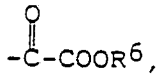, 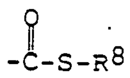, 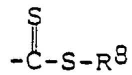, 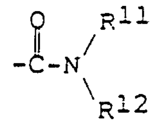 ou 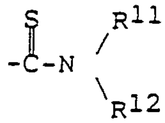 ;

n vaut 0, 1 ou 2 ;

$R^1$, $R^2$, $R^3$ et $R^4$, indépendamment les uns des autres, représentent chacun un hydrogène ou un radical halogéno, alkyle en $C_1$-$C_4$, alcoxy en $C_1$-$C_6$, (alcoxy en $C_1$-$C_6$)-carbonyle, halogénalkyle en $c_1$-$C_2$, nitro, cyano, phénoxy, ou encore un radical phényle pouvant être éventuellement substitué par un radical alcoxy en $C_1$-$C_4$ ou halogéno, deux radicaux $R^1$, $R^2$, $R^3$ ou $R^4$ en position ortho pouvant former ensemble le groupement —CH=CH—CH=CH— ;

$R^5$ est un hydrogène ou un radical alkyle en $C_1$-$C_{12}$, lequel est éventuellement bisubstitué, mais de préférence monosubstitué respectivement par deux ou un des radicaux alcoxy en $C_1$-$C_4$, (alcoxy en $C_1$-$C_4$)-éthoxy, cycloalkyle en $C_3$-$C_6$, benzyloxy, phényle, halogéno, cyano, hydroxyle, oxirannyle, tétrahydrofuryle, (alkyle en $C_1$-$C_4$)amino, di-(alkyle en $C_1$-$C_4$)amino, (alkyle en $C_1$-$C_4$)thio, (alkyle en $C_1$-$C_4$)sulfinyle, (alkyle en $C_1$-$C_4$)sulfonyle, triazolyle ou imidazolyle ; alcényle en $C_3$-$C_6$, halogénalcényle en $C_3$-$C_6$, cycloalkyle en $C_3$-$C_6$, cycloalcényle en $C_5$-$C_6$, alcinyle en $C_3$-$C_6$, benzyle pouvant éventuellement être substitué sur le noyau benzénique par du chlore ou un radical méthyle ; phényle, pouvant être éventuellement substitué jusqu'à deux fois par des radicaux alkyle en $C_1$-$C_4$, nitro, halogéno ou méthoxy ; le groupe

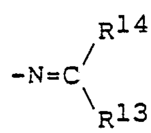

ou un cation d'une base inorganique ou organique ;

$R^6$ est un hydrogène ou un radical alkyle en $C_1$-$C_4$ ou le radical phényle ;

$R^7$ est un hydrogène ou un radical alkyle en $C_1$-$C_6$ éventuellement substitué par un groupe hydroxy ou un groupe alcoxy en $C_1$-$C_3$ ; ou un radical (alkyle en $C_1$-$C_4$)sulfonyle, halogéno-(alkyle en $C_1$-$C_4$)-sulfonyle ; alcényle en $C_3$ ou alcinyle en $C_3$ ;

$R^6$ et $R^7$, avec l'atome d'azote, forment un noyau à 5 ou 6 chaînons, dans lequel un atome de carbone peut être remplacé par un oxygène ou un azote, et est éventuellement substitué par un ou deux groupes méthyle ;

$R^8$ est un hydrogène ou un radical alkyle en $C_1$-$C_6$, phényle, méthylphényle ou halogénophényle, ou un cation d'une base inorganique ou organique ;

$R^9$ est un hydrogène ou un radical alkyle en $C_1$-$C_6$ ;

$R^{10}$ est un radical ayant la signification donnée pour $R^5$, sauf hydrogène, alkylaminoalkyle ou

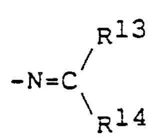

$R^{11}$ est un hydrogène ou un radical alkyle en $C_1$-$C_{12}$, de préférence en $C_1$-$C_6$, lequel peut être éventuellement substitué par un à trois halogènes ou par un groupe alcoxy en $C_1$-$C_4$, nitro, cyano ou phényle, cycloalkyle en $C_3$-$C_6$, alcényle en $C_3$-$C_6$, alcinyle en $C_3$-$C_6$, phényle pouvant éventuellement être substitué par un ou deux groupes halogéno, alkyle en $C_1$-$C_4$, trihalogénométhyle, nitro, alcoxy en $C_1$-$C_4$ ou cyano, ou encore un radical amino, (alkyle en $C_1$-$C_4$)-amino, anilino, di(alkyle en $C_1$-$C_4$)-amino, (alkyle en $C_1$-$C_6$)-carbonyle, trihalogénacétyle, benzoyle, halogénobenzoyle, méthylbenzoyle, phénylsulfonyle ou halogénophénylsulfonyle ;

$R^{12}$ est un radical ayant les significations données pour $R^6$ ;

$R^{11}$ et $R^{12}$, avec l'atome d'azote, forment un noyau à 5 ou 6 chaînons, dans lequel un atome de carbone peut être remplacé par un oxygène ou un azote, et est éventuellement substitué par un ou deux groupes méthyle ;

$R^{13}$ et $R^{14}$, indépendamment l'un de l'autre, sont chacun un hydrogène ou un radical alkyle en $C_1$-

$C_4$, alcoxyalkyle en $C_1$-$C_4$, cycloalkyle en $C_3$-$C_6$ ou le radical phényle, ou encore, avec l'atome de carbone auquel ils sont liés, forment un groupe cycloalkyle en $C_5$ ou $C_6$ ;
ainsi que leurs isomères optiques (si X ≠ Y), leur composé d'addition avec un acide et leurs N-oxydes (si A est N).

2. Procédé pour la préparation de composés de formule I selon la revendication 1, caractérisé en ce qu'on acyle des composés de formule II

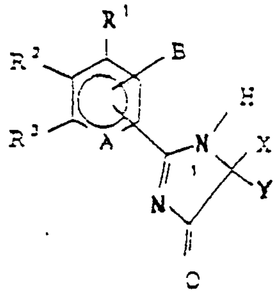

(II)

en position 1 du noyau imidazolinone, avec introduction du radical Z.

3. Utilisation des composés de formule I selon la revendication 1 comme biocides, en particulier herbicides et substances de croissance.

4. Herbicides et substances de croissance, caractérisés en ce qu'ils contiennent un composé de formule générale I selon les revendications 1 et 2.

5. Procédé pour la maîtrise des plantes indésirables, caractérisé en ce qu'on applique sur les surfaces à traiter une quantité efficace d'un composé de formule I selon la revendication 1.

6. Composé de formule

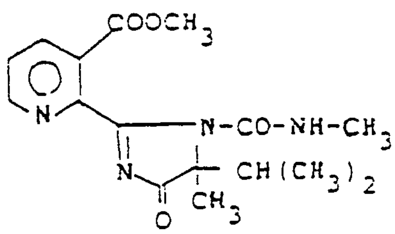

7. Composé de formule

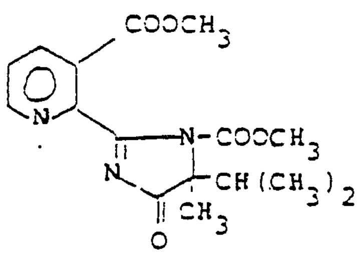

8. Composé de formule

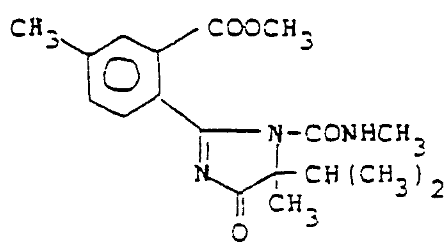

9. Composé de formule

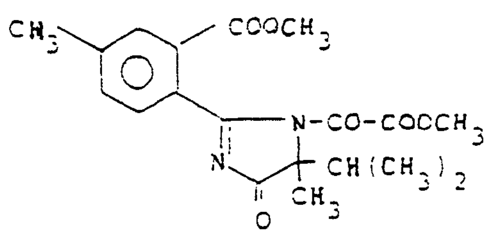

10. Composé de formule

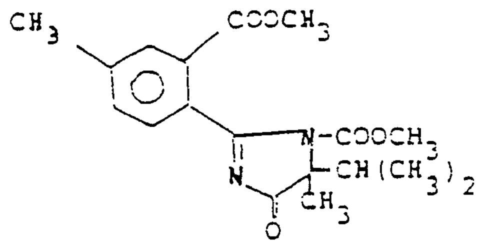

11. Composé de formule

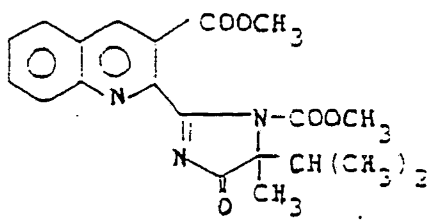

**Revendications** (pour l'Etat contractant AT)

1. Herbicides et substances de croissance, caractérisés en ce qu'ils contiennent des composés de formule générale I

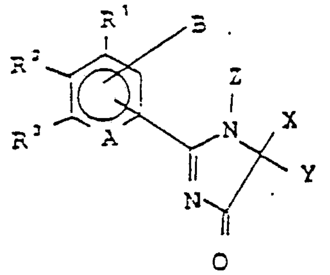

(I)

dans laquelle

A est N ou C—$R^4$ ;

B est

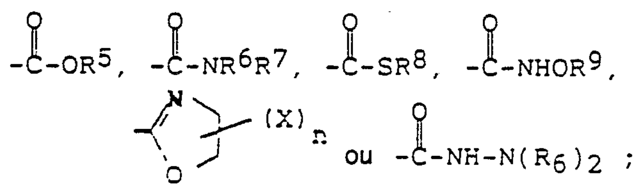

ou ;

X est un radical alkyle en $C_1$-$C_4$ ;

Y est un radical alkyle en $C_1$-$C_6$, cycloalkyle en $C_3$-$C_6$, alcényle en $C_2$-$C_4$, alcinyle en $C_2$-$C_4$, phényle ou benzyle ;

X et Y forment, avec l'atome de carbone auquel ils sont liés, un groupe spirocycloalkyle en $C_3$-$C_6$ éventuellement substitué par un radical $—CH_3$ ;

Z est

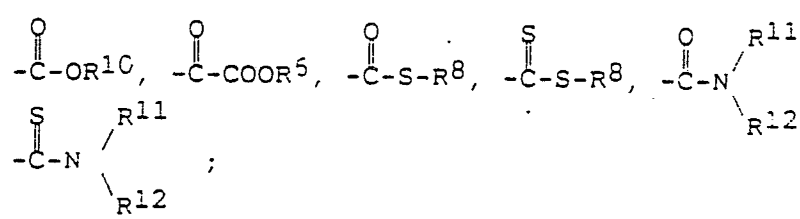

ou ;

n vaut 0, 1 ou 2 ;

$R^1$, $R^2$, $R^3$ et $R^4$, indépendamment les uns des autres, représentent chacun un hydrogène ou un radical halogéno, alkyle en $C_1$-$C_4$, alcoxy en $C_1$-$C_6$, (alcoxy en $C_1$-$C_6$)-carbonyle, halogénalkyle en $C_1$-$C_2$, nitro, cyano, phénoxy, ou encore un radical phényle pouvant être éventuellement substitué par un radical alcoxy en $C_1$-$C_4$ ou halogéno, deux radicaux $R^1$, $R^2$, $R^3$ ou $R^4$ en position ortho pouvant former ensemble le groupement —CH=CH—CH=CH— ;

$R^5$ est un hydrogène ou un radical alkyle en $C_1$-$C_{12}$, lequel est éventuellement bisubstitué, mais de préférence monosubstitué respectivement par deux ou un des radicaux alcoxy en $C_1$-$C_4$, (alcoxy en $C_1$-$C_4$)-éthoxy, cycloalkyle en $C_3$-$C_6$, benzyloxy, phényle, halogéno, cyano, hydroxyle, oxirannyle, tétrahydrofuryle, (alkyle en $C_1$-$C_4$)amino, di-(alkyle en $C_1$-$C_4$)amino, (alkyle en $C_1$-$C_4$)thio, (alkyle en $C_1$-$C_4$)sulfinyle, (alkyle en $C_1$-$C_4$)sulfonyle, triazolyle ou imidazolyle ; alcényle en $C_3$-$C_6$, halogénalcényle en $C_3$-$C_6$, cycloalkyle en $C_3$-$C_6$, cycloalcényle en $C_5$-$C_6$, alcinyle en $C_3$-$C_6$, benzyle pouvant éventuellement être substitué sur le noyau benzénique par du chlore ou un radical méthyle ; phényle, pouvant être éventuellement substitué jusqu'à deux fois par des radicaux alkyle en $C_1$-$C_4$, nitro, halogéno ou méthoxy ; le groupe

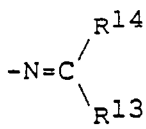

ou un cation d'une base inorganique ou organique ;

$R^6$ est un hydrogène ou un radical alkyle en $C_1$-$C_4$ ou le radical phényle ;

$R^7$ est un hydrogène ou un radical alkyle enh $C_1$-$C_6$ éventuellement substitué par un groupe hydroxy ou un groupe alcoxy en $C_1$-$C_3$ ; ou un radical (alkyle en $C_1$-$C_4$)sulfonyle, halogéno-(alkyle en $C_1$-$C_4$)-sulfonyle ; alcényle en $C_3$ ou alcinyle en $C_3$ ;

$R^6$ et $R^7$, avec l'atome d'azote, forment un noyau à 5 ou 6 chaînons, dans lequel un atome de carbone peut être remplacé par un oxygène ou un azote, et est éventuellement substitué par un ou deux groupes méthyle ;

$R^8$ est un hydrogène ou un radical alkyle en $C_1$-$C_6$, phényle, méthylphényle ou halogénophényle, ou un cation d'une base inorganique ou organique ;

$R^9$ est un hydrogène ou un radical alkyle en $C_1$-$C_6$ ;

$R^{10}$ est un radical ayant la signification donnée pour $R^5$, sauf hydrogène, alkylaminoalkyle ou

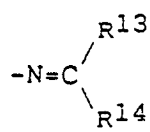

$R^{11}$ est un hydrogène ou un radical alkyle en $C_1$-$C_{12}$, de préference en $C_1$-$C_6$, lequel peut être éventuellement substitué par un à trois halogènes ou par un groupe alcoxy en $C_1$-$C_4$, nitro, cyano ou phényle, cycloalkyle en $C_3$-$C_6$, alcényle en $C_3$-$C_6$, alcinyle en $C_3$-$C_6$, phényle pouvant éventuellement être substitué par un ou deux groupes halogéno, alkyle en $C_1$-$C_4$, trihalogénométhyle, nitro, alcoxy en $C_1$-$C_4$ ou cyano, ou encore un radical amino, (alkyle en $C_1$-$C_4$)-amino, anilino, di(alkyle en $C_1$-$C_4$)-amino, (alkyle en $C_1$-$C_6$)-carbonyle, trihalogénacétyle, benzoyle, halogénobenzoyle, méthylbenzoyle, phénylsulfonyle ou halogénophénylsulfonyle ;

$R^{12}$ est un radical ayant les significations données pour $R^6$ ;

$R^{11}$ et $R^{12}$, avec l'atome d'azote, forment un noyau à 5 ou 6 chaînons, dans lequel un atome de carbone peut être remplacé par un oxygène ou un azote, et est éventuellement substitué par un ou deux groupes méthyle ;

$R^{13}$ et $R^{14}$, indépendamment l'un de l'autre, sont chacun un hydrogène ou un radical alkyle en $C_1$-$C_4$, alcoxyalkyle en $C_1$-$C_4$, cycloalkyle en $C_3$-$C_6$ ou le radical phényle, ou encore, avec l'atome de carbone auquel ils sont liés, forment un groupe cycloalkyle en $C_5$ ou $C_6$ ;
ainsi que leurs isomères optiques (si X ≠ Y), leur composé d'addition avec un acide et leurs N-oxydes (si A est N).

2. Procédé pour la préparation de composés de formule I selon la revendication 1, caractérisé en ce qu'on acyle des composés de formule II

(Voir formule p. 32)

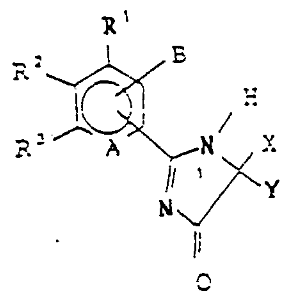

(II)

en position 1 du noyau imidazolinone, avec introduction du radical Z.

3. Utilisation des composés de formule I selon la revendication 1 en tant qu'herbicides et substances de croissance.

4. Procédé pour la maîtrise des plantes indésirables, caractérisé en ce qu'on applique sur les surfaces à traiter une quantité efficace d'un composé de formule I selon la revendication 1.